# EUROPÄISCHE PATENTSCHRIFT

(11) **EP 2 732 850 B1**
(45) Veröffentlichungstag und Bekanntmachung des Hinweises auf die Patenterteilung: **22.04.2020**
(21) Anmeldenummer: 13188214.4
(22) Anmeldetag: 11.10.2013
(51) Int. Cl.: A61Q 11/00, A61K 8/368, A61K 8/27, A61K 8/42, A61K 8/19, A61K 8/97, A61K 8/46

(54) **Feinschaumige Zahncreme mit verbessertem Mundgefühl**
Fine foam toothpaste with improved feeling in the mouth
Crème dentaire finement moussante dotée d'une sensation en bouche améliorée

(30) Priorität: 19.11.2012 DE 102012221078
(43) Veröffentlichungstag der Anmeldung: 21.05.2014
(73) Patentinhaber: Henkel AG & Co. KGaA, 40589 Düsseldorf (DE)
(72) Erfinder: Welß, Thomas, 40591 Düsseldorf (DE); Miehlich, Kristin, 42119 Wuppertal (DE); Giesen, Melanie, 47608 Geldern (DE); Evening, Jennifer, 40472 Düsseldorf (DE)

(56) Entgegenhaltungen:
- EP-A1- 0 348 560
- WO-A1-00/06109
- WO-A2-01/62209
- WO-A2-02/26203
- DE-A1- 10 049 656
- KR-A- 20080 079 776
- US-A- 5 833 956

## Beschreibung

Die Erfindung betrifft eine Zahncreme bzw Zahnpasta, die einen besonders feinen Schaum bildet, dadurch ein verbessertes Mundgefühl bewirkt und die Verfügbarkeit der Wirksubstanzen im Mundraum verbessert.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben Zahnpulvern, die wegen ihrer erhöhten Abrasivität eine untergeordnete Rolle spielen, werden Zahnreinigungsmittel vor allem in Pasten-, Creme- und transluzenter oder transparenter Gelform angeboten. In den letzten Jahren haben auch Liquid- oder Flüssigzahncremes und Mundwässer zunehmend an Bedeutung gewonnen.

Die Kunden bevorzugen dabei Produkte, welche über den Nutzen der Zahnreinigung hinaus weitere positive Effekte bewirken. Dabei wird der Reinigung und Pflege der Zunge und des Zahnfleisches besondere Beachtung geschenkt.

Zahnfleischerkrankungen stellen einen hohen Risikofaktor für die Gesundheit dar. Nach Untersuchungen der Weltgesundheitsorganisation (WHO) leiden über 50 % der erwachsenen Bundesbürger an dringend behandlungsbedürftigen Parodontopathien. Unter Parodontitis versteht man eine durch Bakterien hervorgerufene entzündliche Veränderung des den Zahn umgebenden Gewebes, besonders des Kieferknochens. Eine Parodontitis (= mit Beteiligung der Zahnfleischtaschen und des Knochens) entwickelt sich immer aus einer Zahnfleischentzündung (Gingivitis) und befällt zuerst die der Reinigung am schlechtesten zugänglichen Zahnzwischenräume. Die sich in der Zahnfleischtasche befindlichen Bakterien wirken durch ihre Stoffwechselprodukte destruktiv auf Zahnfleisch, Zahnsubstanz und Knochen ein und verstärken so ständig die bereits bestehende Parodontitis.

Es hat nicht an Versuchen gefehlt, entzündliche Zustände in der Mundhöhle zu bekämpfen, und eine Vielzahl von Agentien wird zu diesem Zweck in Zubereitungen zur Mund- und Zahnpflege und -reinigung eingesetzt. Als antibakterielle Mittel finden beispielsweise Triclosan, Zinnsalze oder Chlorhexidin Verwendung. Der Einsatz dieser Produkte kann jedoch in Einzelfällen mit Nachteilen verbunden sein, die teils geschmacklicher Natur sind, teils ästhetische Gründe haben, da das Produkt und/oder die Zähne durch übermäßigen Gebrauch der Agentien verfärbt werden können. In Einzelfällen kann die Bekämpfung der Bakterien nicht ausreichend sein, um eine Rückgang des entzündlichen Zustandes zu erreichen.

Es besteht daher nach wie vor das Bedürfnis, Zahnfleischentzündungen zu bekämpfen und Wirkstoffe oder Wirkstoffkombinationen dafür bereitzustellen, die frei von den genannten Nachteilen sind.

Die Verwendung von Pflanzenextrakten, welche 4-(3-Ethenyl-3,7-dimethyl-1,6-octadienyl)-phenol enthalten, in Produkten zur oralen Hygiene wird in der JP 2000 327581 beschrieben.

Die Aufgabe der vorliegenden Erfindung bestand darin, Zubereitungen zur Mund- und Zahnpflege und -reinigung bereitzustellen, die Mundgeruch wirksam bekämpfen und gegen Gingivitis und Parodontitis wirksam sind. Dabei sollten in einem Aspekt der Erfindung die Nachteile des Einsatzes von Triclosan, Zinnsalzen oder Chlorhexidin vermieden, deren Einsatzmenge verringert oder auf natürliche antimikrobielle Stoffe zurückgegriffen werden können. In einem weiteren Aspekt der Erfindung sollte die Pflege des Mundraumes durch die Unterstützung der natürlichen Regeneration der Mundschleimhaut und des Zahnfleisches verbessert werden.

Es wurde nun gefunden, daß sich bestimmte Inhaltsstoffe zur Lösung des vorstehend genannten Aufgabenkomplexes eignen.

Gegenstand der vorliegenden Erfindung ist in einer ersten Ausführungsform ein Mund- und Zahnpflege- und -reinigungsmittel, enthaltend - bezogen auf sein Gewicht -
a) 0,5 bis 2,0 Gew.-% anionische(s) Tensid(e),
b) 0,01 bis 1,0 amphotere(s) Tensid(e),
c) 0,1 bis 1,0 Gew.-% Natriumbenzoat
d) 0,001 bis 0,25 Gew.-% mindestens eines Salzes aus der Gruppe der Zink- und/oder Mangansalze,
e) mindestens eine Verbindung aus der Gruppe Panthenol und/oder Panthothensäure und/oder Pantholacton;
f) mindestens einen Kamillenextrakt.

Mund- und Zahnpflegemittel sowie Mund- und Zahnreinigungsmittel im Sinne der Erfindung sind Mund- und Zahnpulver, Mund- und Zahnpasten, flüssige Mund- und Zahncremes sowie Mund- und Zahngele. Bevorzugt geeignet sind Zahnpasten und flüssige Zahnreinigungsmittel. Bevorzugt liegen sie als mehr oder weniger fließfähige oder plastische Zahnpasten vor, wie sie zur Reinigung der Zähne unter Einsatz einer Zahnbürste verwendet werden.

Die erfindungsgemäßen Zusammensetzungen bilden einen besonders feinporigen Schaum, der das Mundgefühl deutlich verbessert und darüber hinaus die Verfügbarkeit der Wirksubstanzen am Zahnfleisch und in der Mundhöhle deutlich verbessert. Im Vergleich zu nicht-erfindungsgemäßen Formulierungen, die einen grobporigeren Schaum bilden, werden entzündliche Zustände des Zahnfleisches und der Mundhöhle deutlich besser bekämpft. Die milde erfindungsgemäße Formulierung führt überdies zu einer Beruhigung schmerzhafter entzündlicher Zustände im Mundraum.

Die erfindungsgemäßen Zusammensetzungen enthalten eine Tensidkombination aus (bezogen auf die Zusammensetzung) 0,5 bis 2,0 Gew.-% anionische(m/n) Tensid(en) und 0,01 bis 1,0 amphotere(m/n) Tensid(en). Siehe hierzu auch WO02/26203, WO01/62210 und DE10049656, die alle Zusammensetzungen mit guter Schaumbildung offenbaren.

Geeignete anionische Tenside sind z. B. lineare Natriumalkylsulfate mit 12-18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12-16 C-Atomen in der linearen Alkylgruppe und 2-6 Glycolethergruppen im Molekül, von linearem Alkan(C₁₂-C₁₈)-sulfonat, von Sulfobernsteinsäuremonoalkyl(C₁₂-C₁₈)-estern, von sulfatisierten Fettsäuremonoglyceriden, sulfatisierten Fettsäurealkanolamiden, Sulfoessigsäurealkyl(C₁₂-C₁₆)-estern, Acylsarcosinen, Acyltauriden und Acylisothionaten mit jeweils 8-18 C-Atomen in der Acylgruppe.

Erfindungsgemäß bevorzugte Mund und Zahnpflege- und -reinigungsmittel sind dadurch gekennzeichnet, daß sie 0,75 bis 1,75 Gew.-%, vorzugsweise 0,85 bis 1,6 Gew.-%, weiter bevorzugt 0,95 bis 1,5 Gew.-%, noch weiter bevorzugt 1,05 bis 1,45 Gew.-% und insbesondere 1,15 bis 1,35 Gew.-% anionische(s) Tensid(e) enthlten.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkyl-sulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen.

Ganz besonders bevorzugte erfindungsgemäße Mittel enthalten Alkylsulfat(e) als anionisches Tensid. Hier sind erfindungsgemäß besonders bevorzugte Mund und Zahnpflege- und - reinigungsmittel sind dadurch gekennzeichnet, daß sie 0,75 bis 1,75 Gew.-%, vorzugsweise 0,85 bis 1,6 Gew.-%, weiter bevorzugt 0,95 bis 1,5 Gew.-%, noch weiter bevorzugt 1,05 bis 1,45 Gew.-% und insbesondere 1,15 bis 1,35 Gew.-% Natriumdodecylsulfat enthalten.

Als weiteres Tensid enthalten die erfindungsgemäßen Zusammensetzungen 0,05 bis 1,0 amphotere(s) Tensid(e). Unter ampholytischen Tensiden und Emulgatoren werden solche oberflächenaktiven Verbindungen verstanden, die außer einer C₈ - C₂₄ - Alkyl- oder -Acylgruppe mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Besonders bevorzugte erfindungsgemäße Mund und Zahnpflege- und -reinigungsmittel sind dadurch gekennzeichnet, daß sie 0,015 bis 0,45 Gew.-%, vorzugsweise 0,02 bis 0,35 Gew.-%, weiter bevorzugt 0,025 bis 0,25 Gew.-%, noch weiter bevorzugt 0,03 bis 0,15 Gew.-% und insbesondere 0,035 bis 0,1 Gew.-% amphotere(s) Tensid(e) enthalten.

Besonders bevorzugte erfindungsgemäße Mund und Zahnpflege- und -reinigungsmittel sind dadurch gekennzeichnet, daß sie amphotere(s) Tensid(e) aus den Gruppen der
- N-Alkylglycine,
- N-Alkylpropionsäuren,
- N-Alkylaminobuttersäuren,
- N-Alkyliminodipropionsäuren,
- N-Hydroxyethyl-N-alkylamidopropylglycine,
- N-Alkyltaurine,
- N-Alkylsarcosine,
- 2-Alkylaminopropionsäuren mit jeweils etwa 8 bis 24 C-Atomen in der Alkylgruppe,
- Alkylaminoessigsäuren mit jeweils etwa 8 bis 24 C-Atomen in der Alkylgruppe,
- N-Kokosalkylaminopropionat,
- Kokosacylaminoethylaminopropionat
- C₁₂ - C₁₈ - Acylsarcosin,
- N-Alkyl-N,N-dimethylammonium-glycinate, beispielsweise Kokosalkyl-dimethylammoniumglycinat,
- N-Acyl-aminopropyl-N,N-dimethylammoniumglycinate, beispielsweise Kokosacylaminopropyl-dimethylammoniumglycinat,
- 2-Alkyl-3-carboxymethyl-3-hydroxyethyl-imidazoline mit jeweils 8 bis 18 C-Atomen in der Alkyl- oder Acylgruppe
- Kokosacylaminoethylhydroxyethylcarboxymethylglycinat
- der unter der INCI-Bezeichnung Cocamidopropyl Betain bekannten Verbindungen,
- der unter der INCI-Bezeichnung Disodium Cocoamphodiacetate bekannten Verbindungen
enthalten.

Besonders bevorzugte Mund und Zahnpflege- und -reinigungsmittel enthalten als amphotere Tenside Betaine der Formel (Bet-I) 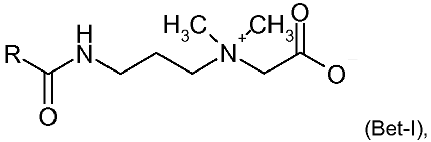 in der R für einen geradkettigen oder verzweigten, gesättigten oder ein- bzw. mehrfach ungesättigten Alkyl- oder Alkenlyrest mit 8 bis 24 Kohlenstoffatomen steht.

Diese Tenside werden nach der INCI-Nomenklatur als Amidopropylbetaine bezeichnet, wobei die Vertreter, die sich von Kokosfettsäuren ableiten, bevorzugt sind und als Cocoamidopropylbetaine bezeichnet werden. Besonders bevorzugt werden erfindungsgemäß Tenside der Formel (Bet-I) eingesetzt, die ein Gemisch der folgenden Vertreter sind:
H₃C-(CH₂)₇-C(O)-NH-(CH₂)₃N⁺(CH₃)₂CH₂COO⁻
H₃C-(CH₂)₉-C(O)-N H-(CH₂)₃N⁺(CH₃)₂C ^{H}2OOO⁻
H₃C-(CH₂)₁₁-C(O)-NH-(CH₂)₃N⁺(CH₃)₂CH₂COO⁻
H₃C-(CH₂)₁₃-C(O)-NH-(CH₂)₃N⁺(CH₃)₂CH₂COO⁻
H₃C-(CH₂)₁₅-C(O)-NH-(CH₂)₃N⁺(CH₃)₂CH₂COO⁻
H₃C-(CH₂)₇-CH=CH-(CH₂)₇-C(O)-NH-(CH₂)₃N⁺(CH₃)₂CH₂COO⁻

Besonders bevorzugt werden Tenside der Formel (Bet-I) innerhalb engerer Mengenbereiche eingesetzt. Hier sind erfindungsgemäße Mund und Zahnpflege- und -reinigungsmittel bevorzugt, die - bezogen auf ihr Gewicht - 0,015 bis 0,45 Gew.-%, vorzugsweise 0,02 bis 0,35 Gew.-%, weiter bevorzugt 0,025 bis 0,25 Gew.-%, noch weiter bevorzugt 0,03 bis 0,15 Gew.-% und insbesondere 0,035 bis 0,1 Gew.-% Cocoamidopropylbetain enthalten.

Für einen besonders feinporigen, stabilen und die Wirksubstanzen besonders effektiv zur Verfügung stellenden Schaum hat es sich als vorteilhaft erwiesen, wenn das Gewichtsverhältnis von anionischen(m/n) Tensid(en) zu amphotere(m/n) Tensid(en) ≥ 1, d.h. wenn die anionischen Tenside im Überschuß zu den amphoteren Tensiden eingesetzt werden.

Erfindungsgemäß besonders bevorzugte Mund und Zahnpflege- und -reinigungsmittel sind dadurch gekennzeichnet, daß das Gewichtsverhältnis von anionische(m/n) Tensid(en) zu amphotere(m/n) Tensid(en) ≥ 2,5:1, vorzugsweise ≥ 5:1, weiter bevorzugt ≥ 6:1 und insbesondere ≥ 7,5:1 beträgt.

Zusammenfassend sind erfindungsgemäße Mund und Zahnpflege- und -reinigungsmittel besonders bevorzugt, die
- 0,75 bis 1,75 Gew.-% Natriumdodecylsulfat und 0,015 bis 0,45 Gew.-% Cocoamidopropylbetain enthalten, wobei das Gewichtsverhältnis von Natriumdodecylsulfat zu Cocoamidopropylbetain ≥ 2,5:1 beträgt;
- 0,85 bis 1,6 Gew.-% Natriumdodecylsulfat und 0,02 bis 0,35 Gew.-% Cocoamidopropylbetain enthalten, wobei das Gewichtsverhältnis von Natriumdodecylsulfat zu Cocoamidopropylbetain ≥ 2,5:1 beträgt;
- 0,95 bis 1,5 Gew.-% Natriumdodecylsulfat und 0,025 bis 0,25 Gew.-% Cocoamidopropylbetain enthalten, wobei das Gewichtsverhältnis von Natriumdodecylsulfat zu Cocoamidopropylbetain ≥ 2,5:1 beträgt;
- 1,05 bis 1,45 Gew.-% Natriumdodecylsulfat und 0,03 bis 0,15 Gew.-% Cocoamidopropylbetain enthalten, wobei das Gewichtsverhältnis von Natriumdodecylsulfat zu Cocoamidopropylbetain ≥ 2,5:1 beträgt;
- 1,15 bis 1,35 Gew.-% Natriumdodecylsulfat und 0,035 bis 0,1 Gew.-% Cocoamidopropylbetain enthalten, wobei das Gewichtsverhältnis von Natriumdodecylsulfat zu Cocoamidopropylbetain ≥ 2,5:1 beträgt.

Weiter bevorzugt sind erfindungsgemäße Mund und Zahnpflege- und -reinigungsmittel, die
- 0,75 bis 1,75 Gew.-% Natriumdodecylsulfat und 0,015 bis 0,45 Gew.-% Cocoamidopropylbetain enthalten, wobei das Gewichtsverhältnis von Natriumdodecylsulfat zu Cocoamidopropylbetain ≥ 5:1 beträgt;
- 0,85 bis 1,6 Gew.-% Natriumdodecylsulfat und 0,02 bis 0,35 Gew.-% Cocoamidopropylbetain enthalten, wobei das Gewichtsverhältnis von Natriumdodecylsulfat zu Cocoamidopropylbetain ≥ 5:1 beträgt;
- 0,95 bis 1,5 Gew.-% Natriumdodecylsulfat und 0,025 bis 0,25 Gew.-% Cocoamidopropylbetain enthalten, wobei das Gewichtsverhältnis von Natriumdodecylsulfat zu Cocoamidopropylbetain ≥ 5:1 beträgt;
- 1,05 bis 1,45 Gew.-% Natriumdodecylsulfat und 0,03 bis 0,15 Gew.-% Cocoamidopropylbetain enthalten, wobei das Gewichtsverhältnis von Natriumdodecylsulfat zu Cocoamidopropylbetain ≥ 5:1 beträgt;
- 1,15 bis 1,35 Gew.-% Natriumdodecylsulfat und 0,035 bis 0,1 Gew.-% Cocoamidopropylbetain enthalten, wobei das Gewichtsverhältnis von Natriumdodecylsulfat zu Cocoamidopropylbetain ≥ 5:1 beträgt.

Noch weiter bevorzugt sind erfindungsgemäße Mund und Zahnpflege- und -reinigungsmittel, die
- 0,75 bis 1,75 Gew.-% Natriumdodecylsulfat und 0,015 bis 0,45 Gew.-% Cocoamidopropylbetain enthalten, wobei das Gewichtsverhältnis von Natriumdodecylsulfat zu Cocoamidopropylbetain ≥ 6:1 beträgt;
- 0,85 bis 1,6 Gew.-% Natriumdodecylsulfat und 0,02 bis 0,35 Gew.-% Cocoamidopropylbetain enthalten, wobei das Gewichtsverhältnis von Natriumdodecylsulfat zu Cocoamidopropylbetain ≥ 6:1 beträgt;
- 0,95 bis 1,5 Gew.-% Natriumdodecylsulfat und 0,025 bis 0,25 Gew.-% Cocoamidopropylbetain enthalten, wobei das Gewichtsverhältnis von Natriumdodecylsulfat zu Cocoamidopropylbetain ≥ 6:1 beträgt;
- 1,05 bis 1,45 Gew.-% Natriumdodecylsulfat und 0,03 bis 0,15 Gew.-% Cocoamidopropylbetain enthalten, wobei das Gewichtsverhältnis von Natriumdodecylsulfat zu Cocoamidopropylbetain ≥ 6:1 beträgt;
- 1,15 bis 1,35 Gew.-% Natriumdodecylsulfat und 0,035 bis 0,1 Gew.-% Cocoamidopropylbetain enthalten, wobei das Gewichtsverhältnis von Natriumdodecylsulfat zu Cocoamidopropylbetain ≥ 6:1 beträgt.

Insbesondere bevorzugt sind erfindungsgemäße Mund und Zahnpflege- und -reinigungsmittel, die
- 0,75 bis 1,75 Gew.-% Natriumdodecylsulfat und 0,015 bis 0,45 Gew.-% Cocoamidopropylbetain enthalten, wobei das Gewichtsverhältnis von Natriumdodecylsulfat zu Cocoamidopropylbetain ≥ 7,5:1 beträgt;
- 0,85 bis 1,6 Gew.-% Natriumdodecylsulfat und 0,02 bis 0,35 Gew.-% Cocoamidopropylbetain enthalten, wobei das Gewichtsverhältnis von Natriumdodecylsulfat zu Cocoamidopropylbetain ≥ 7,5:1 beträgt;
- 0,95 bis 1,5 Gew.-% Natriumdodecylsulfat und 0,025 bis 0,25 Gew.-% Cocoamidopropylbetain enthalten, wobei das Gewichtsverhältnis von Natriumdodecylsulfat zu Cocoamidopropylbetain ≥ 7,5:1 beträgt;
- 1,05 bis 1,45 Gew.-% Natriumdodecylsulfat und 0,03 bis 0,15 Gew.-% Cocoamidopropylbetain enthalten, wobei das Gewichtsverhältnis von Natriumdodecylsulfat zu Cocoamidopropylbetain ≥ 7,5:1 beträgt;
- 1,15 bis 1,35 Gew.-% Natriumdodecylsulfat und 0,035 bis 0,1 Gew.-% Cocoamidopropylbetain enthalten, wobei das Gewichtsverhältnis von Natriumdodecylsulfat zu Cocoamidopropylbetain ≥ 7,5:1 beträgt.

Die erfindungsgemäßen Mittel enthalten als weiteren Inhaltsstoff 0,1 bis 1,0 Gew.-% Natriumbenzoat. Diese Verbindung ist gegen Plaque und/oder Zahnstein wirksam und fördert in Zusammenhang mit dem feinporigen Schaum die Aufnahme der weiteren Wirksubstanzen durch Mundschleimhaut und Zahnfleisch.

Erfindungsgemäß bevorzugte Mund und Zahnpflege- und -reinigungsmittel sind dadurch gekennzeichnet, daß sie 0,125 bis 0,85 Gew.-%, vorzugsweise 0,15 bis 0,75 Gew.-%, weiter bevorzugt 0,175 bis 0,5 Gew.-%, noch weiter bevorzugt 0,2 bis 0,4 Gew.-% und insbesondere 0,25 bis 0,35 Gew.-% Natriumbenzoat enthalten.

Zur Bekämpfung von Plaque eignen sich auch andere antimikrobielle Stoffe. Diese besitzen darüber hinaus eine Wirkung als Konservierungsmittel. In den erfindungsgemäßen Mund und Zahnpflege- und -reinigungsmitteln können zusätzlich zum Natriumbenzoat beispielsweise die auch in Lebensmitteln zugelassenen Konservierungsmittel Sorbinsäure (E 200), Kaliumsorbat (E 202), Calciumsorbat (E 203), Benzoesäure (E 210), Kaliumbenzoat (E 212), Calciumbenzoat (E 213), Ethyl-4-hydroxybenzoat (E 214), Ethyl-4-hydroxybenzoat, Natriumsalz (E 215), Propyl-4-hydroxybenzoat (E 216), Propyl-4-hydroxybenzoat, Natriumsalz (E 217), Methyl-4-hydroxybenzoat (E 218), Methyl-4-hydroxybenzoat, Natriumsalz (E 219), Schwefeldioxid (schweflige Säure), (E 220), Natriumsulfit (E 221), Natriumhydrogensulfit (E 222), Natriumdisulfit (E 223), Kaliumdisulfit (E 224), Calciumsulfit (E 226), Calciumhydrogensulfit (E 227), Kaliumhydrogensulfit (E 228), Biphenyl (E 230), Orthophenylphenol (2-Biphenylol), (E 231), Natriumorthophenylphenolat (E 232), Nisin (E 234), Natamycin (E 235), Ameisensäure (E 236), Natriumformiat (E 237), Calciumformiat (E 238), Hexamethylentetramin (E 239), Dimethyldicarbonat (E 242), Kaliumnitrit (E 249), Natriumnitrit (E 250), Natriumnitrat (E 251), Essigsäure (E 260), Kaliumacetat (E 261), Natriumacetat (E 262), Calciumacetat (E 263), Milchsäure (E 270), Propionsäure (E 280), Natriumpropionat (E 281), Calciumpropionat (E 282), Kaliumpropionat (E 283), Borsäure (E 284), Natriumtetraborat (E 285), Hydroxybernsteinsäure (Äpfelsäure), (E 296), Fumarsäure (E 297), Lysozym (E 1105), eingesetzt werden.

Bevorzugte Stoffe sind ausgewählt aus Alkypyridiniumsalzen, insbesondere Cetylpyridiniumchlorid, p-Hydroxybenzoesäuremethyl-, -ethyl- oder propylester, Natriumsorbat, Bromchlorophen, Triclosan, Phenyl- Salicylsäureester, Biguaniden z. B. Chlorhexidin (1,1'-Hexamethylenbis[5-(4-chlorphenyl)-biguanid), Thymol usw..

Erfindungsgemäß bevorzugte Mund- und Zahnpflege- und -reinigungsmittel sind dadurch gekennzeichnet, daß sie zusätzlich Antiplaque-Wirkstoffe, vorzugsweise Alkypyridiniumsalze, insbesondere Cetylpyridiniumchlorid, p-Hydroxybenzoesäuremethyl-, -ethyl- oder propylester, Natriumsorbat, Bromchlorophen, Triclosan, Hexetidine, Phenyl- Salicylsäureester, Biguanide z. B. Chlorhexidin, Thymol, vorzugsweise in Mengen von 0,05 bis 5 Gew.%, vorzugsweise von 0,10 bis 2,5 Gew.% und insbesondere von 0,30 bis 1,5 Gew.%, jeweils bezogen auf das gesamte Mittel, enthalten.

Als weitere bakterizide Verbindung ist in den erfindungsgemäßen Mitteln 0,001 bis 0,25 Gew.-% mindestens eines Salzes aus der Gruppe der Zink- und/oder Mangansalze enthalten. Diese Salze harmonieren mit dem Tensidsystem und dem Natriumbenzoat und stören weder die Bildung eines feinporigen Schaumes, noch führen sie zu geschmacklichen oder sensorischen Beeinträchtigungen. Besonders bevorzugte Verbindungen sind Salze des Zinks. Erfindungsgemäß bevorzugt können sowohl anorganische als auch organische Salze des Zinks eingesetzt werden. Neben den nicht löslichen anorganischen Zinksalzen, also Salzen, welche eine Löslichkeit unterhalb 100 mg/L (20°C), vorzugsweise unterhalb 10 mg/L (20°C), insbesondere keine Löslichkeit (20°C) aufweisen (z.B. Zinkoxid), sind im Rahmen der vorliegenden Anmeldung die löslichen anorganischen Zinksalze, das heißt Salze, die in Wasser eine Löslichkeit oberhalb 100 mg/L, vorzugsweise oberhalb 500 mg/L, besonders bevorzugt oberhalb 1 g/L und insbesondere oberhalb 5 g/L aufweisen, bevorzugter Bestandteil erfindungsgemäßer Mittel. Zu den bevorzugten löslichen anorganischen Salzen zählen das Zinkbromid, das Zinkchlorid, das Zinkiodid, das Zinknitrat und das Zinksulfat.

Erfindungsgemäß besonders bevorzugte Mittel enthalten dabei mindestens ein Zinksalz aus der nachstehenden Tabelle:

| Zinksalz | Löslichkeit |
|---|---|
| Zinkacetat-Dihydrat | 430 g/l (20°C) |
| Zinkacetylacetonat | 4 g/l (20°C) |
| Zinkbromid | 820 g/l (25°C) |
| Zinkchlorid | 4320 g/l (25°C) |
| Zinkgluconat | 100 g/l (20°C) |
| Zinkhydroxycarbonat | Fast unlöslich (20°C) |
| Zinkiodid | 4500 g/l (20°C) |
| Zinknitrat Hexahydrat | 1843 g/l (20°C) |
| Zinknitrat-Tetrahydrat | Leicht löslich (20°C) |
| Zinkoxid | Unlöslich |
| Zinkstearat | 0,9 mg/l (20°C) |
| Zinksulfat-Heptahydat | 960 g/l (20°C) |
| Zinksulfat-Monohydrat | ∼350 g/l (20°C) |

Das Spektrum der erfindungsgemäß bevorzugten Zinksalze organischer Säuren, vorzugsweise organischer Carbonsäuren, reicht von Salzen die in Wasser nicht löslich sind, also eine Löslichkeit unterhalb 100 mg/L, vorzugsweise unterhalb 10 mg/L, insbesondere keine Löslichkeit aufweisen, bis zu solchen Salzen, die in Wasser eine Löslichkeit oberhalb 100 mg/L, vorzugsweise oberhalb 500 mg/L, besonders bevorzugt oberhalb 1 g/L und insbesondere oberhalb 5 g/L aufweisen (alle Löslichkeiten bei 20°C Wassertemperatur). Zu der ersten Gruppe von Zinksalzen gehören beispielsweise das Zinkcitrat, das Zinlaureat, das Zinkoleat, das Zinkoxalat, das Zinktartrat und das Zinkstearat, zu der Gruppe der löslichen organischen Zinksalze gehören beispielsweise das Zinkacetat, das Zinkacetylacetonat, das Zinkbenzoat, das Zinkformiat, das Zinklactat, das Zinkgluconat, das Zinkvalerat sowie das Zinksalz der p-Toluolsulfonsäure.

Es hat sich gezeigt, daß Zinksulfat ein besonders bevorzugt einzusetzendes Zinksalz ist. Zinksulfat bildet mehrere Hydrate. Das Heptahydrat bildet sich aus gesättigter wäßriger Lösung in Form farbloser, glasglänzender, säulenförmiger, rhombischer Kristalle. Oberhalb 39°C erfolgt Umwandlung zu ZnSO₄ . 6H₂O, und bei 70°C liegt nur noch ZnSO₄ · H₂O vor; das letzte H₂O-Molekül entweicht bei 240°C. Interessanterweise steigt die erfindungsgemäße Wirkung bei den Zinksulfaten mit deren Kristallwassergehalt, so daß das Heptahydrat eine besonders bevorzugte Verbindung ist.

Erfindungsgemäß besonders bevorzugte Mund und Zahnpflege- und -reinigungsmittel sind dadurch gekennzeichnet, daß sie 0,01 bis 0,2 Gew.-%, vorzugsweise 0,02 bis 0,15 Gew.-%, weiter bevorzugt 0,03 bis 0,1 Gew.-%, noch weiter bevorzugt 0,05 bis 0,095 Gew.-% und insbesondere 0,07 bis 0,09 Gew.-% Zinksulfat-Heptahydrat enthalten.

Die erfindungsgemäßen Mittel enthalten als weiteren Inhaltsstoff mindestens eine Verbindung aus der Gruppe Panthenol und/oder Panthothensäure und/oder Pantholacton. Die Gruppe dieser Verbindungen wird auch als Vitamin B₅ bezeichnet. Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat. Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.

Erfindungsgemäß besonders bevorzugte Mund und Zahnpflege- und -reinigungsmittel sind dadurch gekennzeichnet, daß sie 0,125 bis 0,85 Gew.-%, vorzugsweise 0,15 bis 0,75 Gew.-%, weiter bevorzugt 0,175 bis 0,5 Gew.-%, noch weiter bevorzugt 0,2 bis 0,4 Gew.-% und insbesondere 0,25 bis 0,35 Gew.-% Panthenol enthalten.

Als weiteren wesentlichen Inhaltsstoff enthalten die erfindungsgemäßen Mittel mindestens einen Kamillenextrakt. Der Kamillenextrakt beruhigt das Zahnfleisch, beseitigt Zahnfleischirritation und - Blutungen und ist mit dem feinporigen Schaum sehr gut und gleichmäßig applizierbar.

Kamillenextrakt im Sinne der vorliegenden Anmeldung ist ein Stoff oder Stoffgemisch, welches durch Extraktion und teilweises oder völliges Eindampfen der Extraktionslösung gewonnen wurde. Man unterscheidet nach der Beschaffenheit *Trockenextrakte* d.h. bis zur Trockene eingedampfte Extrakte, *Fluidextrakte* d.h. mit Lösungsmitteln so hergestellte Extrakte, daß aus einem Teil Droge höchstens 2 Teile Fluid-Extrakt gewonnen werden, *zähflüssige Extrakte bzw. Dickextrakte,* d.h. Extrakte, bei denen ein Teil des Lösungsmittels verdampft wird.

Die erfindungsgemäß verwendeten Kamillenextrakte werden aus Pflanzen oder Pflanzenteilen durch Extraktion vorzugsweise mit organischen Lösemitteln, Wasser oder Gemischen daraus, gewonnen. Bevorzugt geeignete organische Lösemittel sind Ketone (z.B. Aceton), Ether, Ester, Alkohole oder halogenierte Kohlenwasserstoffe. Besonders bevorzugte Extraktionsmittel sind Wasser und/oder Alkohole. Unter den Alkoholen sind dabei (C₁ bis C₆)- Alkohole, wie Ethanol und Isopropanol und zwar sowohl als alleiniges Extraktionsmittel als auch in Mischung mit Wasser, bevorzugt.

Die Extraktion wird bevorzugt bei einer Temperatur von 25°C bis 90°C durchgeführt.

Üblicherweise werden die Kamillenextrakte durch Extraktion der gesamten Pflanze hergestellt. Es kann aber in einzelnen Fällen auch bevorzugt sein, die Extrakte ausschließlich aus Kamillenblüten und/oder Kamillenblättern herzustellen.

Zur weiteren Steigerung des positiven sensorischen Eindrucks, zur Stabilisierung des feinporigen Schaums und zur weiteren Verbesserung der Wirkung gegen Zahnfleischentzündungen können die erfindungsgemäßen Mittel Bisabolol enthalten.

Hier sind erfindungsgemäße Mund und Zahnpflege- und -reinigungsmittel bevorzugt, die zusätzlich 0,01 bis 1 Gew.-%, vorzugsweise 0,02 bis 0,75 Gew.-%, weiter bevorzugt 0,03 bis 0,5 Gew.-%, noch weiter bevorzugt 0,04 bis 0,25 Gew.-% und insbesondere 0,05 bis 0,1 Gew.-% Bisabolol, vorzugsweise (-)-alpha-Bisabolol 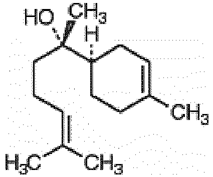 enthalten.

Die erfindungsgemäßen Mund- und Zahnpflege- und -reinigungsmittel enthalten weitere Inhaltsstoffe. Bevorzugt ist hierbei der Einsatz von sogenannten Feuchthaltemitteln, die bei Zahnpasten das Austrocknen verhindern. Bei sogenannten Flüssigzahncremes mit fließfähiger Rheologie dienen diese als Matrix und werden in höheren Mengen eingesetzt. Bei Mundwässern und Mundspülungen dienen diese Alkohole als Konsistenzregler und zusätzliche Süßungsmittel. Hier sind erfindungsgemäße Mund- und Zahnpflege- und -reinigungsmittel bevorzugt, die - bezogen auf ihr Gewicht - 0,5 bis 60 Gew.-%, vorzugsweise 0,75 bis 55 Gew.-%, besonders bevorzugt 1 bis 50 Gew.-% und insbesondere 2 bis 40 Gew.-% mindestens eines mehrwertigen Alkohols aus der Gruppe Sorbit und/oder Glycerin und/oder 1,2-Propylenglycol.-% oder deren Mischungen enthalten.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1) : (0,1-0,5) erwiesen.

Neben Sorbit bzw. Glycerin bzw. 1,2-Propylenglycol eignen sich als weitere mehrwertige Alkohole solche mit mindestens 2 OH-Gruppen, vorzugsweise Mannit, Xylitol, Polyethylenglycol, Polypropylenglycol und deren Mischungen. Unter diesen Verbindungen sind diejenigen mit 2 bis 12 OH-Gruppen und insbesondere diejenigen mit 2, 3, 4, 5, 6 oder 10 OH-Gruppen bevorzugt.

Polyhydroxyverbindungen mit 2 OH-Gruppen sind beispielsweise Glycol (CH₂(OH)CH₂OH) und andere 1,2-Diole wie H-(CH₂)ₙ-CH(OH)CH₂OH mit n = 2, 3, 4, 5, 6, 7, 8, 9, 10, 11, 12, 13, 14, 15, 16, 17, 18, 19, 20. Auch 1,3-Diole wie H-(CH₂)ₙ-CH(OH) CH₂CH₂OH mit n = 1, 2, 3, 4, 5, 6, 7, 8, 9, 10, 11, 12, 13, 14, 15, 16, 17, 18, 19, 20 sind erfindungsgemäß einsetzbar. Die (n,n+1)- bzw. (n,n+2)-Diole mit nicht endständigen OH-Gruppen können ebenfalls eingesetzt werden. Wichtige Vertreter von Polyhydroxyverbindungen mit 2 OH-Gruppen sind auch die Polyethylen- und Polypropylenglycole. Als bevorzugte weitere mehrwertige Alkohole können z. B. Xylit, Propylenglycole, Polyethylenglycole, insbesondere solche mit mittleren Molekulargewichten von 200-800 eingesetzt werden.

Besonders bevorzugt ist der Einsatz von Sorbit, so daß Mittel, die außer Sorbit keine anderen mehrwertigen Alkohole enthalten, besonders bevorzugt sind.
Mund und Zahnpflege- und -reinigungsmittel können darüber hinaus mit besonderem Vorzug Antikaries-Wirkstoffe enthalten. Diese können beispielsweise aus organischen oder anorganischen Fluoriden ausgewählt sein, z. B. aus Natriumfluorid, Kaliumfluorid, Natriummonofluorphosphat und Natriumfluorosilikat. Auch Zinkfluorid, Zinn-(II)-fluorid sind bevorzugt. Bevorzugt sollte eine Menge von 0,01 - 0,2 Gew.-% Fluor in Form der genannten Verbindungen enthalten sein.
Erfindungsgemäße Mund- und Zahnpflege- und -reinigungsmittel, die zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise in Mengen von 0,01 bis 5 Gew.%, vorzugsweise von 0,02 bis 2,5 Gew.% und insbesondere von 0,04 bis 1,1 Gew.%, jeweils bezogen auf das gesamte Mittel, enthalten, sind erfindungsgemäß bevorzugt.

Die Mund- und Zahnpflegemittel, insbesondere die Zahnpasten, können auch die Unempfindlichkeit der Zähne steigernde Substanzen enthalten, beispielsweise Kaliumsalze wie z. B. Kaliumnitrat, Kaliumcitrat, Kaliumchlorid, Kaliumbicarbonat und Kaliumoxalat. Erfindungsgemäß bevorzugte Mund- und Zahnpflege- und -reinigungsmittel sind dadurch gekennzeichnet, daß sie die Unempfindlichkeit der Zähne steigernde Substanzen, vorzugsweise Kaliumsalze, besonders bevorzugt Kaliumnitrat und/oder Kaliumcitrat und/oder Kaliumchlorid und/oder Kaliumbicarbonat und/oder Kaliumoxalat, vorzugsweise in Mengen von 0,5 bis 20 Gew.%, besonders bevorzugt von 1,0 bis 15 Gew.%, weiter bevorzugt von 1,5 bis 5 Gew.-% und insbesondere von 1,75 bis 2,5 Gew.%, jeweils bezogen auf das gesamte Mittel, enthalten.

Der Einsatz von Putzkörpern (Abrasiva) ist ebenfalls bevorzugt. Putzkörper sind amorphe, überwiegend anorganische, weitgehend wasserunlösliche, kleinstteilige Pulver, die keine scharfen Kanten aufweisen. Sie begünstigen in Zahn- und Mundpflegemitteln die Reinigung der Zähne und polieren gleichzeitig die Zahnoberfläche (Poliermittel).

Als Poliermittel eignen sich prinzipiell alle für Zahnpasten bekannten Reibkörper, insbesondere solche, die keine Calciumionen enthalten. Bevorzugt geeignete Poliermittel-komponenten sind daher Kieselsäuren, Aluminiumhydroxid, Aluminiumoxid, Natrium-aluminiumsilikate, organische Polymere oder Gemische solcher Reibkörper.

Calciumhaltige Polierkomponenten wie z.B. Kreide, Calciumpyrophosphat, Dicalcium-phosphatdihydrat können aber in Mengen bis zu 5 Gew.-% - bezogen auf die Gesamtzusammensetzung - enthalten sein.

Der Gesamtgehalt an Poliermitteln liegt vorzugsweise im Bereich von 5 - 50 Gew.-% des Zahnpflegemittels.

Besonders bevorzugt sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die sogenannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident®12 DS (DEGUSSA) erhältlich.

Andere Fällungskieselsäuren dieser Art sind Sident® 8 (DEGUSSA) und Sorbosil® AC 39 (Crosfield Chemicals). Diese Kieselsäuren zeichnen sich durch eine geringere Verdickungswirkung und eine etwas höhere mittlere Teilchengröße von 8 - 14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pa.s aufweisen.

Zahnpasten, die eine deutlich höhere Viskosität von mehr als 100 Pa.s (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, sogenannte Verdickungskieselsäuren mit einer BET-Oberfläche von 150 -250 m²/g zu, z.B. die Handelsprodukte Sipernat 22 LS oder Sipernat® 320 DS.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mund- und Zahnpflege- und -reinigungsmittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ -2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Die angegebenen Mengen beziehen sich auf die Gesamtmenge an Reibkörpern, wobei einzelne Reibkörper vorzugsweise in engeren Mengenbereichen eingesetzt werden. Erfindungsgemäß bevorzugte Mittel enthalten beispielsweise 5 bis 20 Gew.-%, vorzugsweise 8 bis 21 Gew.-%, weiter bevorzugt 9 bis 20 Gew.-% und insbesondere 11 bis 19 Gew.-% Kieselsäure(n). Weiter bevorzugte erfindungsgemäße Mittel sind dadurch gekennzeichnet, daß sie 0,25 bis 2 Gew.-%, vorzugsweise 0,5 bis 1,5 Gew.-% und insbesondere 0,75 bis 1,25 Gew.-% Aluminiumoxid enthalten.

Mund- und Zahnpflege- und reinigungsmittel können z.B. auch Substanzen enthalten, die gegen Plaque und/oder Zahnstein wirksam sind.
Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P ₂O₇, Na₂K₂P₂O₇, Na₂H₂P₂O₇ und K₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.
Erfindungsgemäß bevorzugte Mund- und Zahnpflege- und -reinigungsmittel sind dadurch gekennzeichnet, daß sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.
Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.
Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten.

Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose.

Weitere übliche Hilfs- und Zusatzstoffe für Zahnpasten und Mundwässer oder Mundspüllösungen sind
- Lösungsmittel und Lösungsvermittler, z.B. niedere einwertige oder mehrwertige Alkohole oder Ether, z.B. Ethanol, 1,2-Propylenglycol, Diethylenglycol oder Butyldiglycol
- Pigmente, wie z.B. Titandioxid
- Farbstoffe
- Puffersubstanzen, z.B. primäre, sekundäre oder tertiäre Alkaliphosphate oder Citronensäure-/Na-Citrat
- weitere wundheilende oder entzündungshemmende Stoffe, z.B. Allantoin, Harnstoff, Azulen, Acetylsalicylsäurederivate oder Rhodanid
- weitere Vitamine wie z.B. Ascorbinsäure, Biotin, Tocopherol oder Rutin
- Mineralsalze wie z.B. Mangan-, Zink- oder Magnesiumsalze.

Ein weiterer Gegenstand der vorliegenden Erfindung ist die Verwendung von erfindungsgemäßen Mitteln zur Reinigung von Zähnen mittels manuell betätigter oder elektrischer Zahnbürsten. Bezüglich bevorzugter Ausführungsformen der erfindungsgemäßen Verwendungen gilt mutatis mutandis das zu den erfindungsgemäßen Mitteln Gesagte.

Ein weiterer Gegenstand der vorliegenden Erfindung ist ein Verfahren zur Zahnreinigung, gekennzeichnet durch die Schritte
a) Bereitstellung einer Zahnbürste, deren Bürstenkopf manuell oder elektrisch in Bewegung versetzt werden kann;
b) Applikation von 0,5 bis 5 g eines erfindungsgemäßen Mittels auf den Bürstenkopf,
c) 30- bis 300-sekündiges Zähneputzen mit dem Mittel unter Einsatz des manuell oder elektrisch in Bewegung versetzten Bürstenkopfes.

Bezüglich bevorzugter Ausführungsformen der erfindungsgemäßen Verfahren gilt ebenfalls mutatis mutandis das zu den erfindungsgemäßen Mitteln Gesagte.

## Patentansprüche

1. Mund- und Zahnpflege- und -reinigungsmittel, enthaltend - bezogen auf sein Gewicht -
a) 0,5 bis 2,0 Gew.-% anionische(s) Tensid(e),
b) 0,01 bis 1,0 amphotere(s) Tensid(e),
c) 0,1 bis 1,0 Gew.-% Natriumbenzoat
d) 0,001 bis 0,25 Gew.-% mindestens eines Salzes aus der Gruppe der Zink- und/oder Mangansalze,
e) mindestens eine Verbindung aus der Gruppe Panthenol und/oder Panthothensäure und/oder Pantholacton;
f) mindestens einen Kamillenextrakt.

2. Mund- und Zahnpflege- und -reinigungsmittel nach Anspruch 1, **dadurch gekennzeichnet, daß** es 0,75 bis 1,75 Gew.-%, vorzugsweise 0,85 bis 1,6 Gew.-%, weiter bevorzugt 0,95 bis 1,5 Gew.-%, noch weiter bevorzugt 1,05 bis 1,45 Gew.-% und insbesondere 1,15 bis 1,35 Gew.-% anionische(s) Tensid(e) enthält.

3. Mund und Zahnpflege- und -reinigungsmittel nach einem der Ansprüche 1 oder 2, **dadurch gekennzeichnet, daß** es 0,015 bis 0,45 Gew.-%, vorzugsweise 0,02 bis 0,35 Gew.-%, weiter bevorzugt 0,025 bis 0,25 Gew.-%, noch weiter bevorzugt 0,03 bis 0,15 Gew.-% und insbesondere 0,035 bis 0,1 Gew.-% amphotere(s) Tensid(e) enthält.

4. Mund und Zahnpflege- und -reinigungsmittel nach einem der Ansprüche 1 bis 3, **dadurch gekennzeichnet, daß** das Gewichtsverhältnis von anionische(m/n) Tensid(en) zu amphotere(m/n) Tensid(en) ≥ 2,5:1, vorzugsweise ≥ 5:1, weiter bevorzugt ≥ 6:1 und insbesondere ≥ 7,5:1 beträgt.

5. Mund und Zahnpflege- und -reinigungsmittel nach einem der Ansprüche 1 bis 4, **dadurch gekennzeichnet, daß** es 0,125 bis 0,85 Gew.-%, vorzugsweise 0,15 bis 0,75 Gew.-%, weiter bevorzugt 0,175 bis 0,5 Gew.-%, noch weiter bevorzugt 0,2 bis 0,4 Gew.-% und insbesondere 0,25 bis 0,35 Gew.-% Natriumbenzoat enthält.

6. Mund und Zahnpflege- und -reinigungsmittel nach einem der Ansprüche 1 bis 5, **dadurch gekennzeichnet, daß** es 0,01 bis 0,2 Gew.-%, vorzugsweise 0,02 bis 0,15 Gew.-%, weiter bevorzugt 0,03 bis 0,1 Gew.-%, noch weiter bevorzugt 0,05 bis 0,095 Gew.-% und insbesondere 0,07 bis 0,09 Gew.-% Zinksulfat-Heptahydrat enthält.

7. Mund und Zahnpflege- und -reinigungsmittel nach einem der Ansprüche 1 bis 6, **dadurch gekennzeichnet, daß** es 0,125 bis 0,85 Gew.-%, vorzugsweise 0,15 bis 0,75 Gew.-%, weiter bevorzugt 0,175 bis 0,5 Gew.-%, noch weiter bevorzugt 0,2 bis 0,4 Gew.-% und insbesondere 0,25 bis 0,35 Gew.-% Panthenol enthält.

8. Mund und Zahnpflege- und -reinigungsmittel nach einem der Ansprüche 1 bis 7, **dadurch gekennzeichnet, daß** es 0,01 bis 1 Gew.-%, vorzugsweise 0,02 bis 0,75 Gew.-%, weiter bevorzugt 0,03 bis 0,5 Gew.-%, noch weiter bevorzugt 0,04 bis 0,25 Gew.-% und insbesondere 0,05 bis 0,1 Gew.-% Bisabolol enthält.

9. Verwendung von Mitteln nach einem der Ansprüche 1 bis 8 zur Reinigung von Zähnen mittels manuell betätigter oder elektrischer Zahnbürsten.

10. Verfahren zur Zahnreinigung, **gekennzeichnet durch** die Schritte
a) Bereitstellung einer Zahnbürste, deren Bürstenkopf manuell oder elektrisch in Bewegung versetzt werden kann;
b) Applikation von 0,5 bis 5 g eines Mittels nach einem der Ansprüche 1 bis 8 auf den Bürstenkopf,
c) 30- bis 300-sekündiges Zähneputzen mit dem Mittel nach einem der Ansprüche 1 bis 8 unter Einsatz des manuell oder elektrisch in Bewegung versetzten Bürstenkopfes.

## Claims

1. An oral and dental care and cleaning agent, containing, based on the weight thereof,
a) 0.5 to 2.0 wt.% anionic surfactant(s),
b) 0.01 to 1.0 amphoteric surfactant(s),
c) 0.1 to 1.0 wt.% sodium benzoate,
d) 0.001 to 0.25 wt.% of at least one salt from the group of zinc and/or manganese salts,
e) at least one compound from the group of panthenol and/or pantothenic acid and/or pantolactone;
f) at least one chamomile extract.

2. The oral and dental care and cleaning agent according to claim 1, **characterized in that** it contains 0.75 to 1.75 wt.%, preferably 0.85 to 1.6 wt.%, more preferably 0.95 to 1.5 wt.%, even more preferably 1.05 to 1.45 wt.%, and in particular 1.15 to 1.35 wt.% anionic surfactant(s).

3. The oral and dental care and cleaning agent according to one of claims 1 or 2, **characterized in that** it contains 0.015 to 0.45 wt.%, preferably 0.02 to 0.35 wt.%, more preferably 0.025 to 0.25 wt.%, even more preferably 0.03 to 0.15 wt.%, and in particular 0.035 to 0.1 wt.% amphoteric surfactant(s).

4. The oral and dental care and cleaning agent according to one of claims 1 to 3, **characterized in that** the weight ratio of anionic surfactant(s) to amphoteric surfactant(s) ≥ 2.5:1, preferably ≥ 5:1, more preferably ≥ 6:1 and in particular ≥ 7.5:1.

5. The oral and dental care and cleaning agent according to one of claims 1 to 4, **characterized in that** it contains 0.125 to 0.85 wt.%, preferably 0.15 to 0.75 wt.%, more preferably 0.175 to 0.5 wt.%, even more preferably 0.2 to 0.4 wt.%, and in particular 0.25 to 0.35 wt.% sodium benzoate.

6. The oral and dental care and cleaning agent according to one of claims 1 to 5, **characterized in that** it contains 0.01 to 0.2 wt.%, preferably 0.02 to 0.15 wt.%, more preferably 0.03 to 0.1 wt.%, even more preferably 0.05 to 0.095 wt.%, and in particular 0.07 to 0.09 wt.% zinc sulfate heptahydrate.

7. The oral and dental care and cleaning agent according to one of claims 1 to 6, **characterized in that** it contains 0.125 to 0.85 wt.%, preferably 0.15 to 0.75 wt.%, more preferably 0.175 to 0.5 wt.%, even more preferably 0.2 to 0.4 wt.%, and in particular 0.25 to 0.35 wt.% panthenol.

8. The oral and dental care and cleaning agent according to one of claims 1 to 7, **characterized in that** it contains 0.01 to 1 wt.%, preferably 0.02 to 0.75 wt.%, more preferably 0.03 to 0.5 wt.%, even more preferably 0.04 to 0.25 wt.%, and in particular 0.05 to 0.1 wt.% bisabolol.

9. The use of agents according to one of claims 1 to 8 for cleaning teeth by means of manually operated or electric toothbrushes.

10. A method for cleaning teeth, **characterized by** the steps of:
a) providing a toothbrush, the brush head of which can be set in motion manually or electrically;
b) applying 0.5 to 5 g of an agent according to one of claims 1 to 8 to the brush head;
c) cleaning the teeth with the agent according to one of claims 1 to 8 for 30 to 300 seconds using the brush head that is set in motion manually or electrically.

## Revendications

1. Agent de soin et de nettoyage de la bouche et des dents contenant, par rapport à son poids,
a) de 0,5 à 2,0 % en poids de tensioactif(s) anionique(s),
b) de 0,01 à 1,0 % en poids de tensioactif(s) amphotère(s),
c) de 0,1 à 1,0 % en poids de benzoate de sodium,
d) de 0,001 à 0,25 % en poids d'au moins un sel du groupe des sels de zinc et/ou de manganèse,
e) au moins un composé du groupe panthénol et/ou acide pantothénique et/ou pantolactone ;
f) au moins un extrait de camomille.

2. Agent de soin et de nettoyage de la bouche et des dents selon la revendication 1, **caractérisé en ce qu'**il contient de 0,75 à 1,75 % en poids, de préférence de 0,85 à 1,6 % en poids, de préférence encore de 0,95 à 1,5 % en poids, de manière particulièrement préférée de 1,05 à 1,45 % en poids, et en particulier de 1,15 à 1,35 % en poids de tensioactif(s) anionique(s).

3. Agent de soin et de nettoyage de la bouche et des dents selon l'une des revendications 1 à 2, **caractérisé en ce qu'**il contient de 0,015 à 0,45 % en poids, de préférence de 0,02 à 0,35 % en poids, de préférence encore de 0,025 à 0,25 % en poids, de manière particulièrement préférée de 0,03 à 0,15 % en poids, et en particulier de 0,035 à 0,1 % en poids de tensioactif(s) amphotère(s).

4. Agent de soin et de nettoyage de la bouche et des dents selon l'une des revendications 1 à 3, **caractérisé en ce que** le rapport pondéral entre le(s) tensioactif(s) anionique(s) et le(s) tensioactif(s) amphotère(s) est supérieur ou égal à 2,5:1, de préférence supérieur ou égal à 5:1, de préférence encore supérieur ou égal à 6:1, et en particulier supérieur ou égal à 7,5:1.

5. Agent de soin et de nettoyage de la bouche et des dents selon l'une des revendications 1 à 4, **caractérisé en ce qu'**il contient de 0,125 à 0,85 % en poids, de préférence de 0,15 à 0,75 % en poids, de préférence encore de 0,175 à 0,5 % en poids, de manière particulièrement préférée de 0,2 à 0,4 % en poids, et en particulier de 0,25 à 0,35 % en poids de benzoate de sodium.

6. Agent de soin et de nettoyage de la bouche et des dents selon l'une des revendications 1 à 5, **caractérisé en ce qu'**il contient de 0,01 à 0,2 % en poids, de préférence de 0,02 à 0,15 % en poids, de préférence encore de 0,03 à 0,1 % en poids, de manière particulièrement préférée de 0,05 à 0,095 % en poids, et en particulier de 0,07 à 0,09 % en poids d'heptahydrate de sulfate de zinc.

7. Agent de soin et de nettoyage de la bouche et des dents selon l'une des revendications 1 à 6, **caractérisé en ce qu'**il contient de 0,125 à 0,85 % en poids, de préférence de 0,15 à 0,75 % en poids, de préférence encore de 0,175 à 0,5 % en poids, de manière particulièrement préférée de 0,2 à 0,4 % en poids, et en particulier de 0,25 à 0,35 % en poids de panthénol.

8. Agent de soin et de nettoyage de la bouche et des dents selon l'une des revendications 1 à 7, **caractérisé en ce qu'**il contient de 0,01 à 1 % en poids, de préférence de 0,02 à 0,75 % en poids, de préférence encore de 0,03 à 0,5 % en poids, de manière particulièrement préférée de 0,04 à 0,25 % en poids, et en particulier de 0,05 à 0,1 % en poids de bisabolol.

9. Utilisation d'agents selon l'une des revendications 1 à 8 pour le nettoyage des dents au moyen de brosses à dents manuelles ou électriques.

10. Procédé de nettoyage dentaire, **caractérisé par** les étapes
a) de mise à disposition d'une brosse à dents dont la tête de brosse peut être mise en mouvement manuellement ou électriquement ;
b) d'application de 0,5 à 5 g d'un agent selon l'une des revendications 1 à 8 sur la tête de brosse,
c) de brossage des dents pendant 30 à 300 secondes avec l'agent selon l'une des revendications 1 à 8 en utilisant la tête de brosse mise en mouvement manuellement ou électriquement.
